# EUROPEAN PATENT SPECIFICATION

(11) **EP 2 170 179 B1**
(45) Date of publication and mention of the grant of the patent: **17.06.2015**
(21) Application number: 07786467.6
(22) Date of filing: 31.07.2007
(51) Int. Cl.: A61B 17/16

(54) **CARBON SHAFTED REAMING DEVICE**
FRÄSVORRICHTUNG MIT KOHLENSTOFFSCHAFT
DISPOSITIF D'ALÉSAGE À TIGE DE CARBONE

(43) Date of publication of application: 07.04.2010
(73) Proprietor: Stryker Trauma GmbH, 24232 Schönkirchen/Kiel (DE)
(72) Inventor: AMIROV, Thomas, 24149 Kiel (DE); WIELAND, Manfred, 24146 Kiel (DE)
(74) Representative: Schmitz, Alexander
(86) International application number: PCT/EP2007/006771
(87) International publication number: WO 2009/015672

(56) References cited:
- WO-A-2006/046789
- US-A- 3 751 176
- US-A- 4 751 922
- US-A- 5 116 172
- US-A- 5 488 761
- US-A1- 2005 043 739
- US-A1- 2006 085 004
- US-A1- 2007 015 107
- US-B2- 6 656 195

## Description

### Field of the Invention

The present invention relates to a reaming device, and in particular to a reaming device having a shaft with a carbon fibre reinforced structure.

### Background of the Invention

Intramedullary nailing is the method of choice for the fixation of fractures in long bones, in particular in long extremities. To have a full access to the intramedullary channel, a shaft of a reamer has to be flexible enough in a bending direction to bypass soft tissue and bone curvature, and has also to be rigid enough to convey torsion to the reamer head. Prior art reaming devices have a shaft design consisting of a helix in which residues can be trapped during the reaming procedure, so that the cleaning of the reaming device in hospitals prior to the next usage is complicated, in particular for a sterilisation process. The adequate cleaning of the instrument in hospitals demands a high effort and takes a lot of time: Further, some hospitals are not prepared to clean such critical devices because of the high effort involved.

In some prior art reaming devices, a helix shaft is replaced by a shaft made of so called nitinol, which is a material having a high degree of elasticity (super elasticity) to provide enough flexibility. Nitinol is an akronym for **NI**ckel **TI**tanium **N**aval Ordnance Laboratory. Nitinol is the inter-metallic phase NiTi having a regular cubic crystal structure being different of the structure of titanium or nickel. Nitinol comprises about 55% nickel and about 45% titanium. Owing to the fact that the nitinol shaft is made of a single tube, the cleaning effort in the hospital is less exhausting. However, recent investigations have shown that the nitinol material has a catastrophic failure mode. In particular, some reports have pointed out that some breakages in multiple fragments of the nitinol shaft occurred during the reaming process during the operation process in hospitals. Further, the nitinol material is a very expensive material.

From US 2007/0015107, a root canal instrument having an abrasive coating and method for the production thereof is known, wherein the described root canal instrument has a core of a flexible elastic material having a shape memory, wherein the core furthermore has a coating with abrasive particles, wherein the core is made from a nickel-titanium alloy or from a plastic material, e.g. carbon fibre reinforced plastics material.

CH 668690 describes a probe electrode cable for medical purposes, e.g. electro cardiogram test, using carbon fibre impregnated plastic insulating coating as a cover with a lead coupled to the test equipment.

WO2006/046789 A1 relates to a circular inner surface precision machining tool provided with separable cutting head. The tool includes a body provided with an approximately circular cross-section and being in a thin and long shape. The body includes a metal cover unit and a composite material unit passing through the cover unit in a longitudinal direction. These units may be combined by an adhesive layer between the cover unit and the composite material unit.

US 4,751,922 describes a flexible medullary reamer for shaping the medullary space of bones, wherein the reamer comprises a shaft which may consist of carbon fibre composite and a cutting head which may be attached to the shaft by adhesive bonding.

### Summary of the Invention

It may be seen as an object of the present invention to provide a more reliable reaming device.

The object of the present invention is solved by the subject matter of the independent Claims. Advantageous embodiments thereof are incorporated in the dependent Claims.

According to the invention, a reaming device comprises a rod element, an interface element and a connecting agent, wherein the rod element comprises a first connecting portion having a carbon fibre reinforced structure, wherein the interface element comprises a second connecting portion, wherein the first connecting portion and the second connecting portion are concentrically arranged to each other, and wherein the connecting agent is interposed between the first connecting portion and the second connecting portion.

Thus, a reaming device is provided, which does not have the cleaning problem of the spiral reamer of the prior art, and providing at the same time a more robust material due to the carbon fibre reinforced structure of the rod element. Further, the rod element having a carbon fibre reinforced structure portion is cheaper than the nitinol material of the prior art, and further much more robust. The connecting agent provides a reliable connection between the rod element and the interface element. The interface element may be a coupling element being capable of carrying a reaming tool of a reaming device, but may also be a coupling to a reaming drive of the reaming device. In other words, the interface element may be a coupling on both sides of the reaming device, on the drive input side and the drive output side of the reaming device. The connecting portions, i.e. the first connecting portion and the second connecting portion may be particularly prepared for receiving the connecting agent in order to provide a reliable connection between the rod element and the interface element.

According to an exemplary embodiment of the invention, the first connecting portion is provided on an outer surface of the rod element, and the second connecting portion is provided on an inner surface of the interface element.

Thus, the connection portion of the rod will be at least partially surrounded by the interface element, so that the interface element covers the connecting portion of the rod element. However, the first connecting portion may also be provided on an inner surface of the rod element, and the second connecting portion may be provided on an outer surface of the interface element. In this case, the rod element should be provided with a hole, into which the interface element may be inserted, in particular into which the second connecting portion of the interface element may be inserted. In both of the previously describe cases, the transit from the rod element to the interface element or vice versa may be designed as a smooth transit in order to avoid portions bearing the risk of trapping ablated tissue, which may be problematic with respect to the cleaning process of the reaming device.

According to an exemplary embodiment of the invention, the first connecting portion comprises a first recess, wherein the connecting agent engages into the first recess.

Providing the first connecting portion of the rod element with a recess provides an improved force transmission during the operation of the reaming device, since the force transmission is not limited to the share forces affecting between the connecting agent and the surface of the rod element on the first connecting portion. Moreover, the forces may also be transmitted by the interaction between a protrusion of the connecting agent engaging into the recess and the recess itself.

According to an exemplary embodiment of the invention, the first portion comprises a second recess, wherein the first recess and the second recess are displaced to each other in an axial direction of the rod element.

The provision of a second recess being displaced with respect to the first recess in an axial direction allows to form a further protrusion of the connecting agent so that a force distribution may be improved. The provision of a displacement into an axial direction further distributes the force impact locations to different axial positions, so that the weakening of the rod element in the area of the first connecting portion may be limited in order to avoid a break of the rod element.

According to an exemplary embodiment of the invention, the first portion comprises a third recess, wherein the first recess and the third recess are displaced to each other in a circumferential direction of the rod element.

The provision of several recesses being displaced in a circumferential direction may further improve the distribution of the transmitting forces in order to form an improved contact between the rod element and the interface element.

According to an exemplary embodiment of the invention, the interface element, in particular the second connection portion, comprises a fourth recess, wherein the connecting agent engages into the fourth recess.

The provision of a recess in the interface element provides also an improved force transmission between the connecting agent and the respective interface element.

According to an exemplary embodiment of the invention, the interface element, in particular the second connecting portion comprises a fifth recess, wherein the fourth recess and the fifth recess are displaced in an axial direction of the interface element.

The displacement of the fourth and fifth recess provides an improved force distribution in order to improved the force transmission between the connecting agent and the interface element. The provision of an axial displacement of the recesses may avoid a weakened structure of the interface element and to distribute the force transmission to a plurality of axially distributed locations.

According to an exemplary embodiment of the invention, the interface element comprises a sixth recess, wherein the fourth recess and the sixth recess are displaced in a circumferential direction of the interface element.

The provision of several recesses displaced in a circumferential direction may provide an improved geometry with respect to the force transmission between the connecting agent and the interface element.

It should be noted that a plurality of recesses may be provided which are displaced in both directions, an axial direction and a circumferential direction at the same time. Further, the number of recesses is not limited and may be provided according to the respective need of the application. Further, it should be noted that the connecting agent may engage into the several recesses, in particular also into the second, third, fifth and sixth recess, in order to improve the force transmission between the rod element and the interface element via the connecting agent. It should be further noted, that the recesses in the rod element and the recesses in the interfaces may at least partially correspond to each other with respect to the location of recesses, so that the recesses may face to each other. Thus, the respectively engaging portions of the connecting agent may form a kind of bolting connection between the rod element and the respective interface element. Further, it should be noted that, for example, the third recess may be provided even if there is no second recess, and that a fourth recess may be provided even if there is no first, second or third recess, and so forth. In other words, the recesses may be provided arbitrarily with respect to the need of the respective application.

According to an exemplary embodiment of the invention, at least one of the recesses is formed in a shape of a spherical hole.

The provision of a spherical hole provides the advantage over a cylindrical hole, in that the spherical hole does not provide any sharp chamfer or notch, which sharp chamfer or notch bears the risk of a breakage of the rod element. Thus, by means of a recess in form of a spherical hole a sharp notch or a sharp chamfer may be avoided.

According to an exemplary embodiment of the invention, at least one of the recesses is formed in a shape of a groove, which groove extends into a longitudinal direction of the rod element.

A groove extending in an axial direction has a larger cross-section than a hole, and therefore may provide an improved force transfer between the respective elements.

It should be noted that the groove may have a cross-section of a half circuit in order to avoid sharp notches or sharp chamfers in order to avoid an unintended breakage of the respective elements.

According to the invention, at least one recess forms a through-hole in the interface element.

The provision of a through-hole is much easier to manufacture than a blind hole, in particular when providing such a hole into the inner wall portion of an axial directed bore hole.

According to an exemplary embodiment of the invention, the interface element is adapted to couple a reaming tool to the rod element.

It should be noted that the interface element may also be adapted to couple a drive to the rod element. With providing two interface elements, a first interface element for coupling a reaming tool and a second interface element as a coupling for a drive, the design, in particular the geometry of the connecting agent of both connections between the rod element and the interface element on the reaming tool side and the interface element on the drive side may be designed such that the connecting agent provides a predetermined breaking point on the drive side. Thus, if the driving forces extend over the capability of the intended limited force transmission of the connection between the rod element and the respective interface elements, the predetermined breaking point will be provided on the side of the driving interface element, so that during the operation procedure on or in a human body, the breakage takes place outside the human body, so that no residues of the reaming device remain in the human body. The predetermined breaking point may be provided by, for example, by a reduced number of recesses on the drive side with respect to the reaming tool side.

According to an exemplary embodiment of the invention, the rod element comprises a first conduit extending in a substantially longitudinal direction of the rod element.

The provision of a conduit within the rod element provides the possibility to provide a medical effective agent from the outside to the reaming tool side of the rod element and the respective coupled interface element. On the other hand, the conduit in the rod element provides the possibility to remove the ablated tissue from the reaming tool side to the outside.

According to an exemplary embodiment of the invention, the interface element comprises a second conduit, which conduit being connected to the first conduit of the rod element.

Thus, the second conduit in the interface element corresponds to the first conduit of the rod element, so that the material being transported through the conduit may be transferred from the interface element to the rod element and vice versa. A conduit may be provided for both kinds of interface elements, the interface element as a coupling for a reaming tool and the interface element as a coupling to the drive. It should be noted that the outlet of the conduit of the interface elements may also be provided on the outer wall side, i.e. on the lateral portion of the interface element, in particular when the provision of an agent is desired or the removal of tissue is desired.

According to an exemplary embodiment of the invention, the rod element is made from a carbon fibre composite (CFC).

According to an exemplary embodiment of the invention, the carbon fibres are wound in at least a first layer and a second layer, wherein the direction of the first layer and the second layer are inclined at an angle of substantially plus/minus 45°, respectively, with respect to a longitudinal axis of the rod element.

Thus, the carbon fibres are optimised to have a maximum torsional resistance together with a low bending resistance. It should be noted that also different inclination angles of the direction may be provided, if there is a need to adapt the torsional resistance and the bending resistance as well as the ratio of the torsional resistance and the bending resistance.

According to an exemplary embodiment of the invention, the connecting agent is an adhesive.

The adhesive provides a reliable connection between the rod element and the interface element. It should be noted that for a reaming device an adhesive should be used which is compatible with respect to the human body. An appropriate adhesive should be an adhesive which provides a reliable connection and a bio-compatibility at the same.

According to an exemplary embodiment of the invention, the connecting agent is a thermal hardening adhesive.

A thermal hardening adhesive provides the possibility of a longer manufacturing period, so that when obtaining the correct positioning of the rod element and the interface element to each other, the hardening process may be started, initiated by a heat impact.

According to an exemplary embodiment of the invention, the connecting agent is a multiple component epoxy resin.

Multiple component epoxy resins provide a reliable and strong connection due to the chemical process starting when mixing the multiple components of the epoxy resin or when impacting a heat. Thus, an ageing process or an early binding of the adhesive may be avoided.

According to an exemplary embodiment of the invention, the connecting agent is a third layer of carbon fibre, which third layer is wound around the first layer and the second layer, wherein the third layer is wound into a circumferential direction of the rod element. It should be noted that the first layer and the second layer of the carbon fibre do not have to be separated, and may also constitute an interwoven structure. However, if the carbon fibres are inclined with respect to a solely circumferential direction, the stability of the structure may be weakened, so that the winding of a third carbon fibre layer in a circumferential direction provides a sufficient stable structure, in particular when the third carbon layer serves as a connecting agent. The third carbon layer may serve as a connecting agent in cases, the rod element and the interface element are connected by a press fitting, which does not need an adhesive for a reliable connection between the rod element and the interface element.

It should be noted that the above features may also be combined. The combination of the above features may also lead to synergetic effects, even if not explicitly described in detail.

These and other aspects of the present invention will become apparent from and elucidated with reference to the embodiments described hereinafter.

### Brief Description of the Drawings

Exemplary embodiments of the present invention will be described in the following with reference to the following drawings.
Fig. 1 illustrates an exemplary embodiment of a rod element, a connecting agent and an interface element, which interface element may serve as a coupling for a reaming tool.
Fig. 2 illustrates an embodiment not according to the invention of the rod element, the connecting agent and an interface element, which interface element may serve as a coupling to a drive.
Figs. 3a and 3b illustrate the separated components of a reaming device according to an embodiment not forming part of the invention.
Figs. 4a and 4b illustrate the mounted components of Figs. 3a and 3b.
Figs. 5a, 5b, 5c and 5d illustrate a rod element having a connecting portion according to an embodiment not forming part of the invention.
Figs. 6a, 6b, 6c and 6d illustrate a rod element having a connection portion according to an embodiment not forming part of the invention.
Figs. 7a, 7b, 7c, 7d, 7e and 7f illustrate an interface element serving as a coupling for a reaming tool according to an exemplary embodiment.
Figs. 8a, 8b, 8c and 8d illustrate an interface element serving as a coupling to a drive according to an embodiment not forming part of the invention.
Figs. 9a, 9b, 9c and 9d illustrate an interface element serving as a coupling to a drive according to an embodiment not forming part of the invention.

### Detailed Description of Exemplary Embodiments

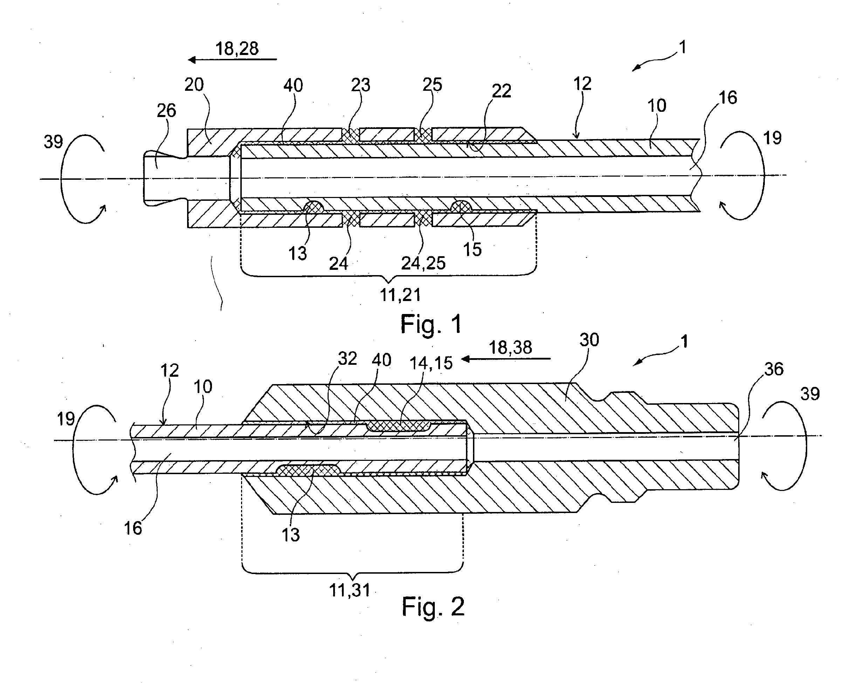

Fig. 1 illustrates an exemplary embodiment of the coupling of the rod element 10 and the interface element 20 by means of a connecting agent 40. The rod element 10 may be provided with a conduit 16. The rod element according to the illustrated embodiment comprises a first recess 13 and a third recess 15, which are formed as spherical holes. In this embodiment, the holes are blind holes, so that the sealing of the conduit 16 may be upheld in the region of the holes or recesses. It should be noted the recesses may also be through holes. The rod element 10 is provided with a connection portion 11. The interface element 20 in this embodiment is provided with a fourth, fifth and sixth recess 23, 24, 25. The recesses may be provided on displaced locations with respect to the longitudinal axis 18 of the rod element, which corresponds to the longitudinal axis of the interface element 28 in this embodiment.

As can be seen, recess 23 is displaced in the longitudinal direction 28 to the recess 25. The same is valid for the recesses 13 and 15, which are displaced to each other with respect to the longitudinal axis 18 of the rod element 10. The recesses of the interface element 20 are provided as bore holes. It should be noted that the recesses may also be displaced with respect to a circumferential direction of the interface element 39, as can be seen from the recesses 23 and 24, which are displaced by about 180°, however any other degree of displacement may be applied according to need. Although Fig. 1 does not illustrate the circumferential displacement of recesses of the rod element 10 into a circumferential direction 19, it should be noted that also recesses may be provided, which are displaced with respect to the circumferential direction 19 of the rod element 10. The interface element 20 is provided with a second connecting portion 21 which corresponds to the first connecting portion 11 of the rod element 10. A connecting agent 40 is provided between the concentrically arranged first connecting portion 11 of the rod element 10 and the second connecting portion 21 of the interfacing element 20. In the illustrated embodiment, the connecting agent 40 engages into the recesses 13, 15, 23, 24 and 25, so that an improved force transfer between the rod element 10 and the interface element 20 may be provided. However, it should be noted that if the force transfer between the rod element 10 and the interface element 20 is sufficient, the contact agent 40 does not have to engage into the recesses, even if the recesses are provided. In this case, it should be noted, that the recesses may also be left out.

Fig. 2 illustrates a reaming device 1 having a rod element 10 and an interface element 30, wherein the rod element 10 and the interface element 30 are concentrically arranged at least in the first connecting portion 11 and the second connecting portion 31. Fig. 2 illustrates recesses on the outer surface 12 of the rod element 10, which are formed as grooves extending into the longitudinal direction 18 of the rod element 10. The longitudinal direction 18 of the rod element 10 and the longitudinal direction 38 of the interface element 30 correspond to each other in the present embodiment. The recesses 13, 14 and 15 provided on the surface 12 of the rod element 10 are displaced with respect to the longitudinal direction 18 of the rod element 10, as well as they are displaced with respect to the circumferential direction 19 of the rod element 10. The rod element 10 and the interface element 30 are concentrically arranged in the first connecting portion 11 of the rod element 10 and the second connecting portion 31 of the interface element 30. The illustrated rod element 10 comprises a conduit 16 which corresponds to a conduit 36 of the interface element 30, so that a transport of a medical agent or tissue may be carried out. A connecting agent 40 is provided between the rod element 10 and the interface element 30, wherein the connecting agent 40 may engage into the recesses 13, 14 and 15. Thus, an improved force transfer may take place between the interface element 30 and the rod element 10.

If, for example, leaving out the fourth, fifth and sixth recess on the driving side interface element 30 of Fig. 2 a predetermined breaking point may be provided, since the force transfer between the interface element 30 and the rod element 10 may be limited, so that if extending the applied forces, the connection by the connecting agent 40 between the rod element 10 and the interface element 30 intendedly will break, so that a break of the rod element 10 as such and a break between the rod element 10 and the reaming tool sided interface element 20 of Fig. 1 may be avoided, so that the predetermined breaking point is provided outside the human body for every operation situation.

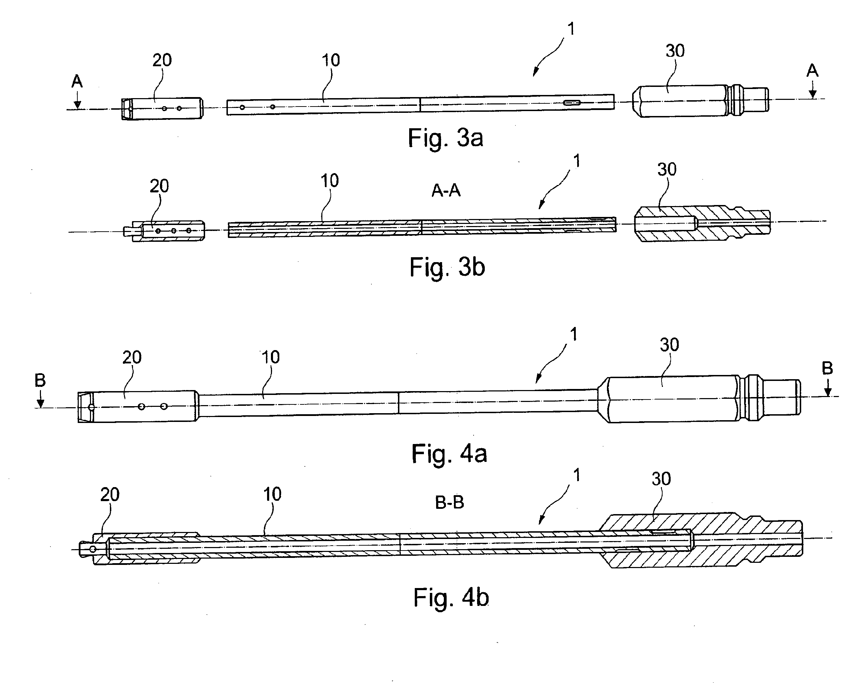

Fig. 3a and Fig. 3b illustrate as a side view (Fig. 3a) and a cross-sectional view (Fig. 3b) of the several components of a reaming device 1 having a rod element 10 and two interface elements 20, 30. The connecting agent 40 is not illustrated in Figs. 3a and 3b. As can be seen from Fig. 3b, the provision of a conduit in all components 10, 20, 30 provides a connection to deliver any medical agent or to remove tissue from the reaming tool (not shown).

Fig. 4a and Fig. 4b illustrate the assembled reaming device 1 having a rod element 10 and two interface elements 20, 30. As can be seen from the cross-sectional view in Fig. 4b, the rod element 10 and the interface elements 20, 30 are concentrically arranged such that the connecting portion of the interface elements 20, 30 cover the respective connecting portions of the rod element 10.

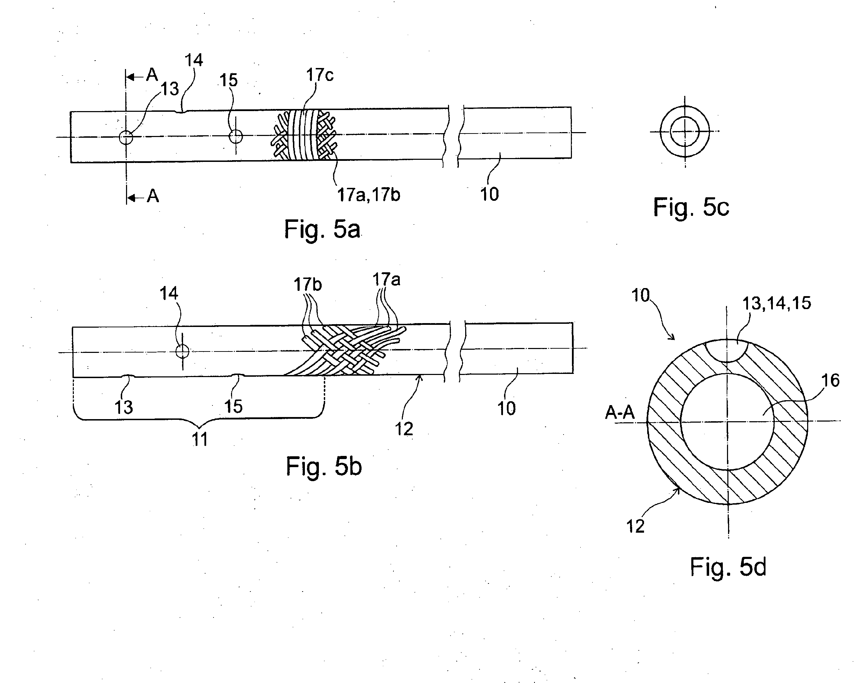

Figs. 5a, 5b, 5c and 5d illustrate the rod element 10, and in particular the connecting portion 11 of the rod element 10. In the connecting portion 11 of the rod element 10, there may be provided recesses 13, 14 and 15, wherein the recesses may be displaced with respect to the longitudinal direction, as can be seen in Fig. 5a, or may be displaced in a circumferential direction, as can be seen from Fig. 5b, which is rotated by 90° over the illustration of Fig. 5a.

Further, Fig. 5a illustrates an exemplary embodiment of the carbon fibre layers, wherein the first and second carbon fibre layer 17a, 17b may be provided as an interwoven structure, as can be seen from Fig. 5b. The interwoven structure may be surrounded by a third layer 17c, which is wound in the circumferential direction of the rod element 10. The circumferential winding in particular is relevant if applying a press fitting between the interface elements 20, 30 and the rod element 10. Fig. 5c illustrates a cross-sectional view along the cut A-A, wherein Fig. 5d illustrates an enlarged cross-sectional view of Fig. 5c showing some more details. Fig. 5d illustrates an exemplary embodiment of a recess 13, 14, 15, which is formed as a spherical hole. It should be noted that a spherical hole should also be understood as a hole formed by a part of a sphere, as can be seen from Fig. 5d. Further, the recesses 13, 14, 15, 23, 24, 25, 33, 34, 35 may have any other form, e.g. a cylindrical form or a form without sharp notches. This however is not limited to spherical holes.

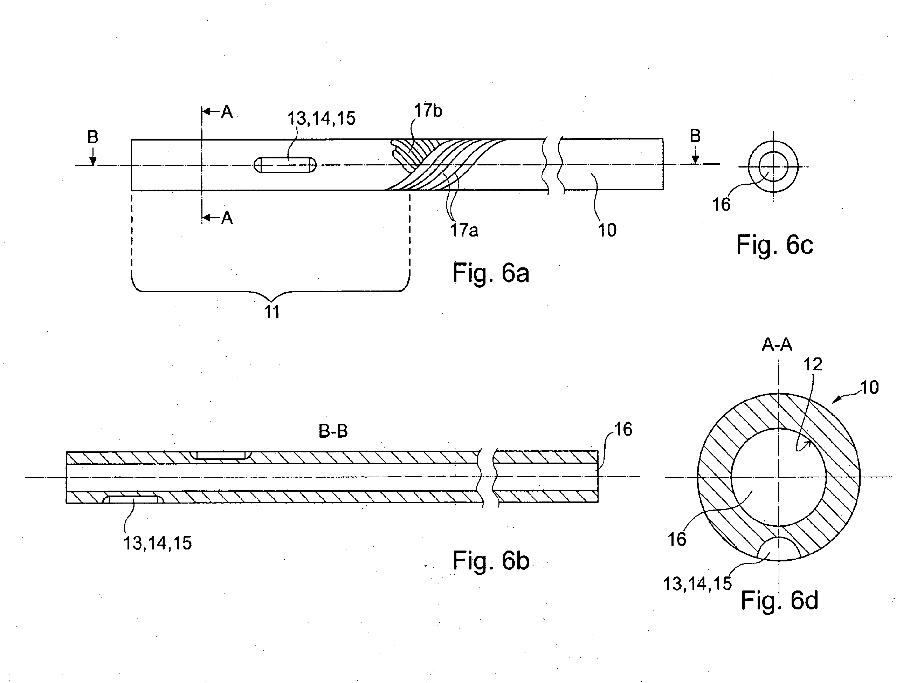

Figs. 6a, 6b, 6c and 6d illustrate a further embodiment not according to the invention, illustrating recesses 13, 14, 15 on the surface side of the rod element 10, which recesses are formed as grooves into a longitudinal direction of the rod element 10. The grooves may be displaced with respect to the longitudinal axis of the rod element 10, as can be seen from Fig. 6b, as well as displaced into a circumferential direction, as can be seen from Fig. 6a, which illustrates a view of the rod element of Fig. 6b being rotated by 90°. Fig. 6c illustrates a cross-sectional view of Fig. 6a, and Fig. 6d illustrates an enlarged cross-sectional view of the rod element shown in Fig. 6c. As can be seen from Fig. 6d, the recesses 13, 14 and 15 may have a cross-section in form of a circle sector in order to avoid sharp notches or sharp chamfers in order to avoid a damage of the rod element 10 when transferring forces.

Fig. 6a illustrates further a first layer 17a and a second layer 17b of the carbon fibres, wherein the carbon fibres in the embodiment of Fig. 6a are wound in separate layers. It should be noted that also any other arrangement of the first and second layer 17a and 17b may be provided, in particular any other woven pattern may be used, where it is appropriate and necessary for the respective application according to need. The number of layers is however not limited to a first and second layer, and may be also a multi layer structure.

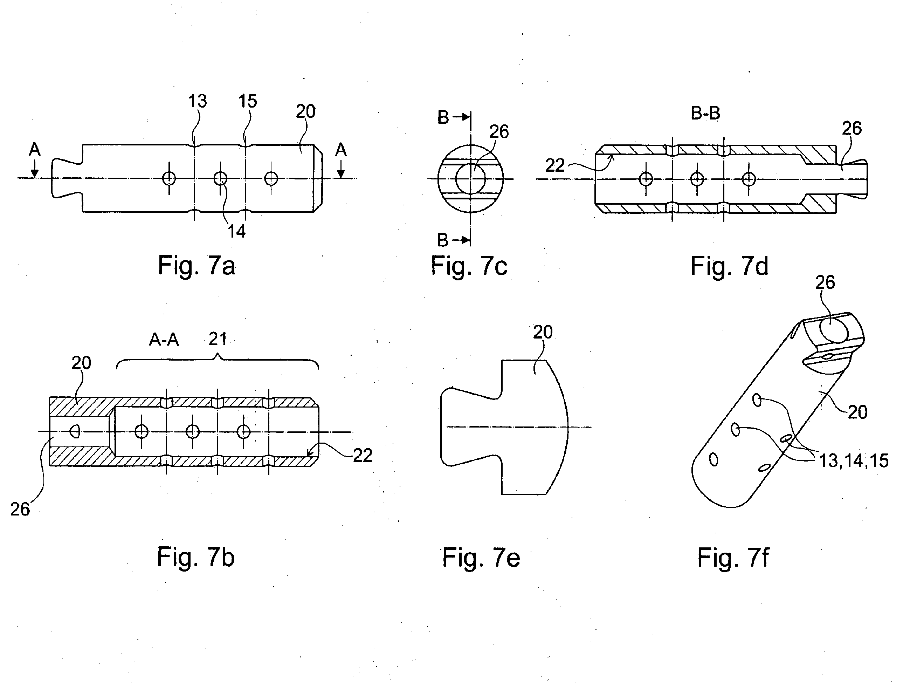

Figs. 7a, 7b, 7c, 7d, 7e and 7f illustrate an interface element 20, which is adapted to couple a reaming tool. The coupling of the reaming tool takes place at the head of the interface element 20, a detail of which is illustrated in Fig. 7e. The second connecting portion 21 of the interface element 20 may be provided with a plurality of recesses 13, 14 and 15, which may be displaced into an axial direction as well as a circumferential direction, as can be seen from Fig. 7a and the corresponding cross-sectional view of Fig. 7b. The interface element 20 may also be provided with a conduit 26 which may provide a connection between the conduit of a rod element 16 (not shown in any of the Figs. 7a, 7b, 7c, 7d and 7f) to a conduit of a reaming tool (also not shown).

Fig. 7c illustrates a top view of the illustration of Fig. 7a. Fig. 7d illustrates a cross-sectional view of the interface element 20, rotated by 90° over the illustration of Fig. 7b. Fig. 7f illustrates a three-dimensional view of the interface element 20.

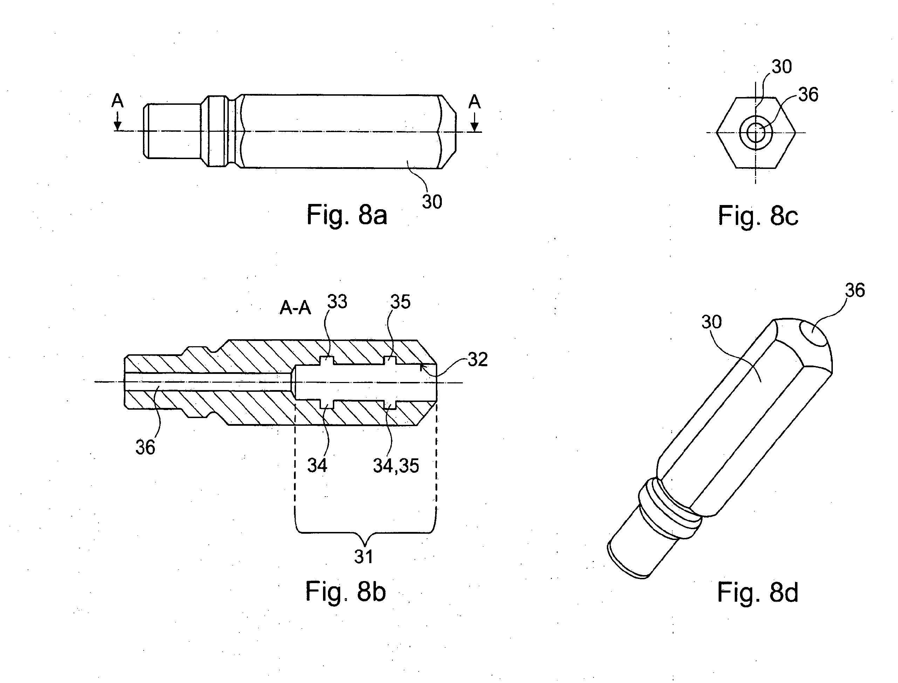

Figs. 8a, 8b, 8c and 8d illustrate a further embodiment of an interface element, however this interface element is adapted to couple a drive for driving the reaming device. Fig. 8a illustrates a side view of the exemplary interface element 30. Fig. 8c illustrates a top view of the interface element 30 shown in Fig. 8a. Fig. 8b illustrates a cross-sectional view of the interface element 30 of Fig. 8a, wherein the interface element 30 is also provided with a conduit 36 into a longitudinal direction. The connecting portion 31 comprises a plurality of recesses 33, 34, 35, which recesses may be provided as blind holes as well as through-holes (not shown). The recesses may be formed as cylindrical holes as well as spherical holes (not shown). The recesses 33, 34, 35 may be provided on the inner surface 32 of a bore hole, which bore hole is adapted to receive the connecting portion 11 of the rod element 10. Fig. 8d illustrates a three-dimensional view of the interface element 30 according to an exemplary embodiment.

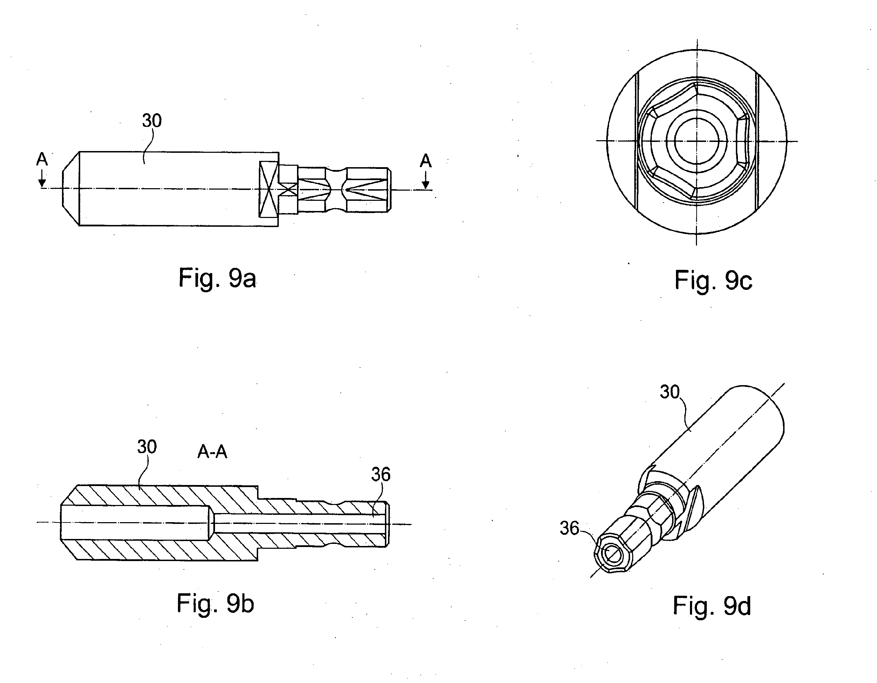

Figs. 9a, 9b, 9c and 9d illustrate a further embodiment not according to the invention of an interface element 30, which is adapted to be coupled to a drive. Fig. 9a illustrates a side view, Fig. 9c illustrates an enlarged top view, and Fig. 9b illustrates a cross-sectional view of the interface element 30. Fig. 9d illustrates a three-dimensional view of the interface element 30.

The interface elements of Figs. 8a, 8b, 8c and 8d differ from the interface elements of Figs. 9a, 9b, 9c and 9d in that they provide a different coupling geometry for a drive, which may be specified with respect to the supplier of the drive unit. Thus, it should be noted that the design of the coupling geometry may be modified with respect to the drive unit to be coupled to the interface element 30.

It should be noted that the term 'comprising' does not exclude other elements and the 'a' or 'an' does not exclude a plurality. Also elements described in association with the different embodiments may be combined.

It should be noted that the reference signs in the Claims shall not be construed as limiting the scope of the Claims.

## Claims

1. Reaming device comprising:
a rod element (10);
an interface element (20, 30) for coupling a reaming tool to a shaft; and
a connecting agent (40);
wherein the rod element (10) comprises a first connecting portion (11) having a carbon fibre reinforced structure;
wherein the interface element (20, 30) comprises a second connecting portion (21, 31);
wherein the first connecting portion (11) and the second connecting portion (21, 31) are concentrically arranged to each other; and
wherein the connecting agent (40) is interposed between the first connecting portion (11) and the second connecting portion (21, 31),
**characterized in that** the second connecting portion comprises a recess (23, 24, 25) called fourth recess, wherein the recess (23, 24, 25) forms a through hole in the interface element (20, 30) and wherein the connecting agent engages into the recess (23, 24, 25).

2. Reaming device of claim 1, wherein the first connecting portion (11) is provided on an outer surface (12) of the rod element (10), and the second connecting portion (21, 31) is provided on an inner surface (22, 32) of the interface element (20, 30).

3. Reaming device of any of claims 1 and 2, wherein the first connecting portion (11) comprises a further recess (13) called first recess, wherein the connecting agent (40) engages into the first recess (13).

4. Reaming device of claim 3, wherein the first connecting portion (11) comprises a further recess (14) called second recess, wherein the first recess (13) and the second recess (14) are displaced to each other in an axial direction (18) of the rod element (10), wherein the connecting agent (40) engages into the second recess (14).

5. Reaming device of any of claims 3 and 4, wherein the first connecting portion (11) comprises a further recess (15) called third recess, wherein the first recess (13) and the third recess (15) are displaced to each other in a circumferential direction (19) of the rod element, wherein the connecting agent (40) engages into the third recess (15).

6. Reaming device of claim 1, wherein the second connecting portion (21, 31) comprises a further recess (24, 34) called fifth recess, wherein the fourth recess (23, 33) and the fifth recess (24, 34) are displaced in an axial direction (28, 38) of the interface element (20, 30), wherein the connecting agent (40) engages into the fifth recess (24, 34).

7. Reaming device of any of claims 1 and 6, wherein the second connecting portion (21, 31) comprises a further recess (25, 35) called sixth recess, wherein the forth recess (23, 33) and the sixth recess (25, 35) are displaced in a circumferential direction (19) of the interface element (20, 30), wherein the connecting agent (40) engages into the sixth recess (25, 35).

8. Reaming device of any of claims 2 to 7, wherein at least one of the recesses (13, 14, 15; 23, 24, 25; 33, 34, 35) is formed in a shape of a spherical hole.

9. Reaming device of any of claims 2 to 7, wherein at least one of the recesses (13, 14, 15; 23, 24, 25; 33, 34, 35) is formed in a shape of a groove, which groove extends into a longitudinal direction (18) of the rod element (10).

10. Reaming device of any of claims 1 to 9, wherein the interface element (20, 30) is adapted to couple a reaming tool to the rod element (10).

11. Reaming device of any of claims 1 to 10, wherein the rod element (10) comprises a first conduit (16) extending in a substantially longitudinal direction (18) of the rod element (10).

12. Reaming device of claim 11, wherein the interface element (20, 30) comprises a second conduit (26, 36), the second conduit (26, 36) being connected to the first conduit (16) of the rod element (16).

13. Reaming device of any of claims 1 to 12, wherein the rod element (10) is made from a carbon fibre composite.

14. Reaming device of any of claims 1 to 13, wherein the carbon fibres (17) are wound in at least a first layer (17a) and a second layer (17b), the direction of the first layer (17a) and the second layer (17b) are inclined at an angle of substantially plus/minus 45°, respectively, with respect to a longitudinal axis (18) of the rod element (10).

15. Reaming device of any of claims 1 to 14, wherein the connecting agent (40) is an adhesive.

16. Reaming device of claim 15, wherein the connecting agent (40) is a thermal hardening adhesive.

17. Reaming device of any of claims 1 to 16, wherein the connecting agent (40) is a multiple component epoxy resin.

## Patentansprüche

1. Fräsvorrichtung, Folgendes umfassend:
ein Stabelement (10);
ein Zwischenelement (20, 30) zum Verbinden eines Fräswerkzeugs mit einer Welle; und
ein Befestigungsmittel (40);
wobei das Stabelement (10) einen ersten Verbindungsabschnitt (11) mit einer kohlefaserverstärkten Struktur aufweist;
wobei das Zwischenelement (20, 30) einen zweiten Verbindungsabschnitt (21, 31) aufweist;
wobei der erste Verbindungsabschnitt (11) und der zweite Verbindungsabschnitt (21, 31) konzentrisch zueinander angeordnet sind; und
wobei das Befestigungsmittel (40) zwischen dem ersten Verbindungsabschnitt (11) und dem zweiten Verbindungsabschnitt (21, 31) angeordnet ist,
**dadurch gekennzeichnet, dass** der zweite Verbindungsabschnitt eine Aussparung (23, 24, 25), vierte Aussparung genannt, aufweist, wobei die Aussparung (23, 24, 25) eine Durchgangsbohrung im Zwischenelement (20, 30) ausbildet und wobei das Befestigungsmittel in die Aussparung (23, 24, 25) eingreift.

2. Fräsvorrichtung nach Anspruch 1, wobei der erste Verbindungsabschnitt (11) an einer Außenfläche (12) des Stabelements (10) bereitgestellt ist und der zweite Verbindungsabschnitt (21, 31) an einer Innenfläche (22, 32) des Zwischenelements (20, 30) bereitgestellt ist.

3. Fräsvorrichtung nach Anspruch 1 oder 2, wobei der erste Verbindungsabschnitt (11) eine weitere Aussparung (13), erste Aussparung genannt, aufweist, wobei das Befestigungsmittel (40) in die erste Aussparung (13) eingreift.

4. Fräsvorrichtung nach Anspruch 3, wobei der erste Verbindungsabschnitt (11) eine weitere Aussparung (14), zweite Aussparung genannt, aufweist, wobei die erste Aussparung (13) und die zweite Aussparung (14) in einer axialen Richtung (18) des Stabelements (10) voneinander versetzt sind, wobei das Befestigungsmittel (40) in die zweite Aussparung (14) eingreift.

5. Fräsvorrichtung nach Anspruch 3 oder 4, wobei der erste Verbindungsabschnitt (11) eine weitere Aussparung (15), dritte Aussparung genannt, aufweist, wobei die erste Aussparung (13) und die dritte Aussparung (15) in einer Umfangsrichtung (19) des Stabelements voneinander versetzt sind, wobei das Befestigungsmittel (40) in die dritte Aussparung (15) eingreift.

6. Fräsvorrichtung nach Anspruch 1, wobei der zweite Verbindungsabschnitt (21, 31) eine weitere Aussparung (24, 34), fünfte Aussparung genannt, aufweist, wobei die vierte Aussparung (23, 33) und die fünfte Aussparung (24, 34) in einer axialen Richtung (28, 38) des Zwischenelements (20, 30) voneinander versetzt sind, wobei das Befestigungsmittel (40) in die fünfte Aussparung (24, 34) eingreift.

7. Fräsvorrichtung nach Anspruch 1 oder 6, wobei der zweite Verbindungsabschnitt (21, 31) eine weitere Aussparung (25, 35), sechste Aussparung genannt, aufweist, wobei die vierte Aussparung (23, 33) und die sechste Aussparung (25, 35) in einer Umfangsrichtung (19) des Zwischenelements (20, 30) voneinander versetzt sind, wobei das Befestigungsmittel (40) in die sechste Aussparung (25, 35) eingreift.

8. Fräsvorrichtung nach einem der Ansprüche 2 bis 7, wobei wenigstens eine der Aussparungen (13, 14, 15, 23, 24, 25, 33, 34, 35) die Form eines runden Lochs aufweist.

9. Fräsvorrichtung nach einem der Ansprüche 2 bis 7, wobei wenigstens eine der Aussparungen (13, 14, 15, 23, 24, 25, 33, 34, 35) die Form einer Nut aufweist, wobei die Nut in einer Längsrichtung (18) des Stabelements (10) verläuft.

10. Fräsvorrichtung nach einem der Ansprüche 1 bis 9, wobei das Zwischenelement (20, 30) angepasst ist, ein Fräswerkzeug mit dem Stabelement (10) zu verbinden.

11. Fräsvorrichtung nach einem der Ansprüche 1 bis 10, wobei das Stabelement (10) einen ersten Kanal (16) aufweist, der sich im Wesentlichen in einer Längsrichtung (18) des Stabelements (10) erstreckt.

12. Fräsvorrichtung nach Anspruch 11, wobei das Zwischenelement (20, 30) einen zweiten Kanal (26, 36) aufweist, wobei der zweite Kanal (26, 36) mit dem ersten Kanal (16) des Stabelements (16) verbunden ist.

13. Fräsvorrichtung nach einem der Ansprüche 1 bis 12, wobei das Stabelement (10) aus einem Kohlefaserverbundstoff hergestellt ist.

14. Fräsvorrichtung nach einem der Ansprüche 1 bis 13, wobei die Kohlefasern (17) in wenigstens einer ersten Schicht (17a) und einer zweiten Schicht (17b) gewunden sind, wobei die Richtungen der ersten Schicht (17a) und der zweiten Schicht (17b) in einem Winkel von im Wesentlichen plus/minus 45° zueinander bzw. im Verhältnis zu einer Längsachse (18) des Stabelements (10) geneigt ist.

15. Fräsvorrichtung nach einem der Ansprüche 1 bis 14, wobei das Befestigungsmittel (40) ein Klebstoff ist.

16. Fräsvorrichtung nach Anspruch 15, wobei das Befestigungsmittel (40) ein thermisch aushärtender Klebstoff ist.

17. Fräsvorrichtung nach einem der Ansprüche 1-16, wobei das Befestigungsmittel (40) ein Mehrkomponenten-Epoxidharz ist.

## Revendications

1. Dispositif d'alésage comportant :
un élément de tige (10),
un élément d'interface (20, 30) pour coupler un outil d'alésage à un arbre, et
un agent de liaison (40),
dans lequel l'élément de tige (10) comporte une première partie de liaison (11) ayant une structure renforcée par des fibres de carbone,
dans lequel l'élément d'interface (20, 30) comporte une seconde partie de liaison (21, 31),
dans lequel la première partie de liaison (11) et la seconde partie de liaison (21, 31) sont agencées de manière concentrique l'une par rapport à l'autre, et
dans lequel l'agent de liaison (40) est intercalé entre la première partie de liaison (11) et la seconde partie de liaison (21, 31),
**caractérisé en ce que** la seconde partie de liaison comporte un évidement (23, 24, 25) appelé quatrième évidement, dans lequel l'évidement (23, 24, 25) forme un trou traversant dans l'élément d'interface (20, 30) et dans lequel l'agent de liaison s'engage dans l'évidement (23, 24, 25).

2. Dispositif d'alésage selon la revendication 1, dans lequel la première partie de liaison (11) est agencée sur une surface extérieure (12) de l'élément de tige (10), et la seconde partie de liaison (21, 31) est agencée sur une surface intérieure (22, 32) de l'élément d'interface (20, 30).

3. Dispositif d'alésage selon l'une quelconque des revendications 1 et 2, dans lequel la première partie de liaison (11) comporte un évidement supplémentaire (13) appelé premier évidement, dans lequel l'agent de liaison (40) s'engage dans le premier évidement (13).

4. Dispositif d'alésage selon la revendication 3, dans lequel la première partie de liaison (11) comporte un évidement supplémentaire (14) appelé deuxième évidement, dans lequel le premier évidement (13) et le deuxième évidement (14) sont déplacés l'un par rapport à l'autre dans une direction axiale (18) de l'élément de tige (10), dans lequel l'agent de liaison (40) s'engage dans le deuxième évidement (14).

5. Dispositif d'alésage selon l'une quelconque des revendications 3 et 4, dans lequel la première partie de liaison (11) comporte un évidement supplémentaire (15) appelé troisième évidement, dans lequel le premier évidement (13) et le troisième évidement (15) sont déplacés l'un par rapport à l'autre dans une direction circonférentielle (19) de l'élément de tige, dans lequel l'agent de liaison (40) s'engage dans le troisième évidement (15).

6. Dispositif d'alésage selon la revendication 1, dans lequel la seconde partie de liaison (21, 31) comporte un évidement supplémentaire (24, 34) appelé cinquième évidement, dans lequel le quatrième évidement (23, 33) et le cinquième évidement (24, 34) sont déplacés dans une direction axiale (28, 38) de l'élément d'interface (20, 30), dans lequel l'élément de liaison (40) s'engage dans le cinquième évidement (24, 34).

7. Dispositif d'alésage selon l'une quelconque des revendications 1 et 6, dans lequel la seconde partie de liaison (21, 31) comporte un évidement supplémentaire (25, 35) appelé sixième évidement, dans lequel le quatrième évidement (23, 33) et le sixième évidement (25, 35) sont déplacés dans une direction circonférentielle (19) de l'élément d'interface (20, 30), dans lequel l'agent de liaison (40) s'engage dans le sixième évidement (25, 35).

8. Dispositif d'alésage selon l'une quelconque des revendications 2 à 7, dans lequel au moins l'un des évidements (13, 14, 15 ; 23, 24, 25 ; 33, 34, 35) a une forme de trou sphérique.

9. Dispositif d'alésage selon l'une quelconque des revendications 2 à 7, dans lequel au moins un des évidements (13, 14, 15 ; 23, 24, 25 ; 33, 34, 35) a une forme de gorge, laquelle gorge s'étend dans une direction longitudinale (18) de l'élément de tige (10).

10. Dispositif d'alésage selon l'une quelconque des revendications 1 à 9, dans lequel l'élément d'interface (20, 30) est adapté pour coupler un outil l'alésage à l'élément de tige (10).

11. Dispositif d'alésage selon l'une quelconque des revendications 1 à 10, dans lequel l'élément de tige (10) comporte un premier conduit (16) s'étendant dans une direction sensiblement longitudinale (18) de l'élément de tige (10).

12. Dispositif d'alésage selon la revendication 11, dans lequel l'élément d'interface (20, 30) comporte un second conduit (26, 36), le second conduit (26, 36) étant relié au premier conduit (16) de l'élément de tige (10).

13. Dispositif d'alésage selon l'une quelconque des revendications 1 à 12, dans lequel l'élément de tige (10) est fabriqué à partir d'un composite en fibres de carbone.

14. Dispositif d'alésage selon l'une quelconque des revendications 1 à 13, dans lequel les fibres de carbone (17) sont enroulées en au moins une première couche (17a) et une seconde couche (17b), les directions de la première couche (17a) et de la seconde couche (17b) sont inclinées à un angle sensiblement égal à plus/moins 45°, respectivement, par rapport à un axe longitudinal (18) de l'élément de tige (10).

15. Dispositif d'alésage selon l'une quelconque des revendications 1 à 14, dans lequel l'agent de liaison (40) est un adhésif.

16. Dispositif d'alésage selon la revendication 15, dans lequel l'agent de liaison (40) est un adhésif de durcissement thermique.

17. Dispositif d'alésage selon l'une quelconque des revendications 1 à 16, dans lequel l'agent de liaison (40) est une résine époxyde à composants multiples.
